# EUROPEAN PATENT APPLICATION

(11) **EP 2 233 110 A1**
(43) Date of publication of application: **29.09.2010**
(21) Application number: 09004200.3
(22) Date of filing: 24.03.2009
(51) Int. Cl.: A61C 19/04

(54) **Methods and apparatus to determine distances for use in dentistry**

(71) Applicant: orangedental GmbH & Co. KG, 88400 Biberach (DE)
(72) Inventor: Piernitzki, Bernhard, 19089 Kobande (DE); Laxhuber, Ludwig, Dr., 8221 Herrsching (DE)
(74) Representative: Zwicker, Jörk

(57) **Abstract**

The invention relates to the field of medical methods and equipment and in preferred embodiments to equipment and methods used in dentistry. In particular it relates to a computer-aided method for automatically recording the distance between a probe of a medical device and a reference point. The invention also provides a computer storage medium comprising a computer-readable program code to carry out said method, an apparatus for automatically recording the insertion depth of said probe into a body cavity and uses thereof.

## Description

### FIELD OF THE INVENTION

The invention relates to the field of medical methods and equipment and in preferred embodiments to equipment and methods used in dentistry. In particular it relates to a computer-aided method for automatically recording the distance between a probe of a medical device and a reference point. The invention also provides a computer storage medium comprising a computer-readable program code to carry out said method, an apparatus for automatically recording the insertion depth of said probe into a body cavity and uses thereof.

### BACKGROUND OF THE INVENTION

Routine medical inspections frequently require the accurate determination of the dimensions of a body cavity, organ, or parts thereof. Hereby measurements are taken in one, two and/or three dimensions, to determine e.g. the depth, width and breadth of said cavity or organ. The value of accurate medical measurements lies in their utility for diagnostic but also therapeutic purposes. For example, it is routinely necessary to determine the dimensions of a tumor growing on or in a tissue e.g. an organ. In another example from the field of dentistry, the accurate depth of periodontal pockets and gingival grooves surrounding the teeth is required for computing a periodontal disease index (PDI). The periodontal disease index (PDI) is an index used for estimating the degree of periodontitis (see also: Ramfjord SP, J. Periodontol. 1967; 38: 602-610, incorporated herein by reference) and can give an accurate assessment of a person's periodontal status. It is useful for assessing the need for treatment and for evaluation of the results following treatment.

Medical instruments comprised in the art and capable of measuring the dimensions of a body cavity, organ, or parts thereof always require an action of the operator to activate the recording of the respective measurement. Such action may involve, e.g. the pressing of a button or the reading of a scale or the reading and noting down of individual measurements. However, these required actions that the operator has to perform will occasionally result in a mechanical shifting of the device or in a vibration which can lead to inaccurate measurements. Also, if a multiplicity of measurements and recordings are required then the repeated action, e.g. the repeated pressing of a button, e.t.c., becomes exhaustive such that the duration of the diagnostic and/or therapeutic treatment is increased and will thus be less well tolerated by the patient. A further disadvantage is that in one diagnosis or treatment session only a limited number of measurements can be taken.

Thus, the object of the present invention is to provide for an improved method and apparatus for examining the dimensions of a patient's organs, body cavities and parts thereof and which allows the acquisition of more accurate data in a shorter time span.

### SUMMARY OF THE INVENTION

To solve the aforementioned problems and to provide means for obtaining fast and accurate results during the measurement of the dimensions of tissue, organs, body cavities, diseased body parts and so on, the invention provides in a first aspect A computer-aided method for automatically recording the distance between a probe of a medical device and a reference point comprising the steps of:
a) providing a medical device comprising a probe;
b) moving said probe under manual control with respect to a reference point;
c) automatically determining
   (i) if said probe has essentially not moved with respect to said reference point during a predefined time interval and/or
   (ii) if the directionality of movement of said probe with respect to said reference point was reversed during a predefined time interval;
   and
d) automatically recording the distance between said probe and said reference point if at least one of the conditions in step (c) are fulfilled.

In a further aspect the invention provides a computer storage medium comprising a computer-readable program code for causing a data processing system to carry out the computer-aided method of the invention.

The invention also relates to an apparatus for automatically recording the insertion depth of said probe into a body cavity, wherein the apparatus comprises a computer system, means to carry out the computer-aided method of any of the invention and said medical device including said probe.

Also provided is the use of the computer-aided method according to the invention, or the apparatus according to the invention, for determining the size of a body cavity, of an organ or parts thereof.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, GenBank Accession Number sequence submissions etc.), whether *supra* or *infra*, is hereby incorporated by reference in its entirety. Nothing herein is to be construed as an admission that the invention is not entitled to antedate such disclosure by virtue of prior invention.

A "patient" in the sense of the present application may be any animal to which the computer implemented method of the invention and/or the apparatus of the invention can advantageously be applied. As such, a "patient" may be any animal. In more preferred embodiments, a "patient" is a mammal. In especially preferred embodiments, a patient is a horse, a donkey, cattle, a sheep, a goat, a pig, a dog, a cat, or, in particular, a human.

As used herein "movement" refers to a distance travelled and/or to the velocity of movement, i.e. the displacement over a time interval, wherein the distance/displacement can comprise any positive (forward movement) or negative value (reverse movement) or can also be zero. Thus, determining "movement" can also mean determining zero velocity, i.e. the expandable and/or retractable probe stands still. In preferred embodiments, "movement" as used herein also comprises positive and negative acceleration.

The term "dielectric" refers to a material which essentially does not conduct electrical current, i.e. it refers to any insulating substance a plurality of which are known in the art and also described below.

To provide an improved method for determining the dimensions of organs, body cavities, diseased tissues and parts thereof, in particular for determining and automatically recording the depth of body cavities, the present invention provides in a first aspect a computer-aided method for automatically recording the distance between a probe of a medical device and a point comprising the steps of:
a) providing a medical device comprising a probe;
b) moving said probe under manual control with respect to a reference point;
c) automatically determining
   (i) if said probe has essentially not moved with respect to said reference point during a predefined time interval and/or
   (ii) if the directionality of movement of said probe with respect to said reference point was reversed during a predefined time interval;
   and
d) automatically recording the distance between said probe and said reference point if at least one of the conditions in step (c) are fulfilled.

In a preferred embodiment, the probe can be a) can be extended and/or retracted from said medical device.

The reference point enables the optical, mechanical and/or electric measurement of the distance between the reference point and said probe. By definition the reference point is not located on the extendable and/or retractable probe. In a preferred embodiment, the localization of said reference point is defined by a reference probe. In one embodiment, the location of said reference point has previously been defined such as in step a) or prior to step a). For example, the reference point can be a predefined point on the medical device. The reference point can in preferred embodiments also be the opening of a body cavity such as the opening of an access puncture in the skin of a patient undergoing an endoscopic treatment or the opening of a root canal of a tooth, the opening of a periodontal pocket and of a gingival groove. The reference probe is preferably a part of or stably affixed to the medical device used in the method. Also any object that is stationary with respect to the movement of the probe can serve as reference probe.

In a particularly preferred embodiment of the method of the invention the method is a computer-aided method for automatically recording the relative position of an elongated probe of a medical device with respect to said device comprising the steps of:
a) providing a medical device comprising an elongated probe that can be extended and/or retracted through an opening in said medical device;
b) extending and/or retracting said elongated probe under manual control;
c) automatically determining
   (i) if said probe has essentially not moved with respect to said opening during a predefined time interval and/or
   (ii) if the directionality of movement of said probe with respect to said opening was reversed during a predefined time interval;
   and
d) automatically recording the distance between the tip of said elongated probe and said opening if at least one of the conditions in step (c) are fulfilled and if said distance is a positive value.

Further preferred embodiments of the reference point and the reference probe are described herein below.

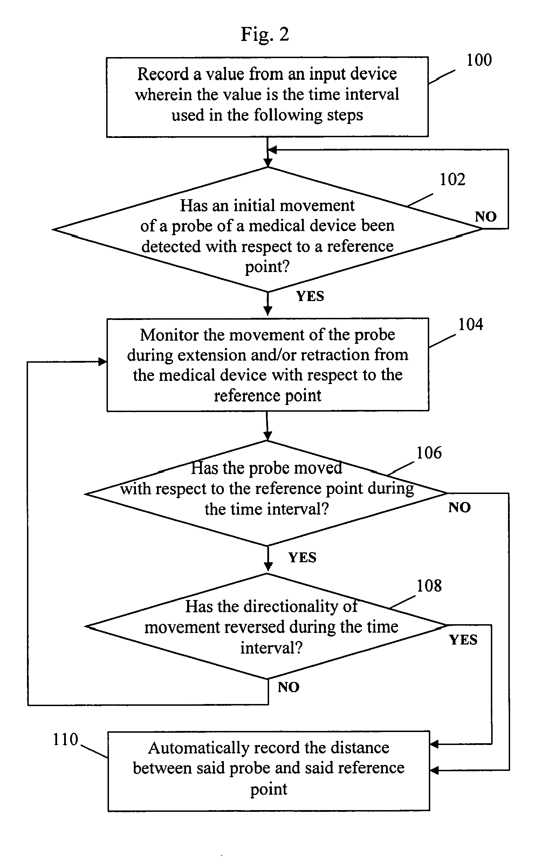

The time interval used in the computer-aided method of the invention is preferably predefined to equal a value of between 0.001 and 1 seconds and preferably of about 0.3, 0.4 or 0.5 seconds. It is to be understood that the time interval does not limit the number of distance or movement measurements that are determined by the method in step b) and/or c). Preferably, at least 1, 2, 3, 4, 5, 6, 7, 8, 9 or at least 10 and most preferably at least 5 distance measurements are made in step b) and/or c) of the method per second (see also item 104 of figure 2 below).

In step d) of the method said distance is automatically recorded (see also item 110 of figure 2 below). The recording can e.g. occur in form of a print out or in electronic form, for example the distance value is automatically stored on a storage device such as a hard disk, RAM, ROM, an EEPROM (for example flash memory) and/or EPROM memory for later use.

In a preferred embodiment of the method of the invention, the distance in step d) is not recorded if the probe has not left its initial resting position, i.e. if the probe has not departed from its location with respect to the reference point that it had before the probe was extended or retracted under manual control in step b). Thus, it is preferred that step c) of the computer-aided method of the invention is carried out during or after step b). Similarly, it is preferred that steps c) and d) are not carried out before step b) (see also item 102 of figure 2 in this context). In the computer-aided method and the apparatus (see below) of the present invention can, for example comprise or carry out a further step which is detecting an initial movement of the probe with respect to a reference point, e.g. a retraction and/or extension of the probe. As used herein the phrase "initial movement" refers to a movement of the probe which is greater than a predefined threshold value as described in detail below. If in said optional extra step said initial movement has been detected, the method then continues with steps b), c) and d). Preferably, in step b) all further movements of the probe are monitored until at least one of the conditions comprised in step c) is true. In one embodiment, if at least one of the conditions comprised in step c) is true then step d) as described herein is carried out. In another embodiment, during step b) a plurality of distance measurements between the probe and the reference point are recorded together with the time points that said recordings were made in. From the distance and time point data recorded during step b) of the preferred embodiment of the method, the velocity, preferably average velocity and/or the displacement, preferably maximal displacement and, thus, the movement of the probe with respect to the reference point can be computed. Thus, steps c) and d) of the method can be carried out at a later time preferably in an *in silico* analysis of the distance and time point data recorded in step b).

In a further embodiment of the method of the invention comprises a step c) wherein the movement of the probe with respect to said reference point and/or the distance in step d) is measured with an optical, mechanical or electrical means.

Preferred optical means comprise the optical recording, e.g. using a video camera, of both the probe or part of the probe and the reference point. Using such optical means, the distance can be computed that the probe or part of the probe has moved with respect to the reference point. In one example, the probe is an elongated probe comprising optically detectable markings which indicate how far the probe has been extended from the medical device, which in this preferred embodiment is the reference point used in the method. Many alternative optical methods are comprised in the art and known by the average skilled person, see e.g. United States patent US 5,244,387.

Preferred mechanical means comprise, e.g. the use of a spring-action or the use of a rack and pinion which are coupled to a display device as is well known in the art of mechanical engineering.

The movement, preferably extension and retraction of the probe is under manual control. Thus, preferred electrical means for determining the distance and, thus, also the movement of the probe with respect to a reference point include monitoring of the manual operation of the probe by the operator. If the operator directly handles the probe, e.g. by pulling and pushing the elongated probe along or through said medical device, this movement can be recorded by e.g. optical, electrical and/or mechanical means, e.g. a small motor is mechanically coupled to the movement generating a current which correlates with the velocity of movement of the probe. If in preferred embodiments of the method the movement of the probe is motorized and the motor activity is under manual control, then the movement of the probe can be monitored by determining the turning speed of the motor or the current which is directed to the motor. Further preferred electrical means comprise, e.g. the generation of a repeated acoustic signal wherein the pitch, duration and/or frequency of the acoustic signal correlates to the velocity of movement of the probe with respect to the medical device. Electrical means useful to measure periodontal pocket depths are also described in e.g. United States patent US 4,791,940.

In step b) said probe is moved under manual control with respect to a reference point. In this context, the moving of said probe means spatial displacement of said probe. The movement can in preferred embodiments occur by extending and/or retracting said probe from said medical device as also mentioned above. As used herein the terms "extending" and "retracting" have the regular meaning associated with these verbs. Extension and retraction can also occur by way of expansion (e.g. telescope mechanic) and shrinkage. In preferred embodiments, the elongated probe is essentially not compressible or stretchable in its length axis, it is, however, in preferred embodiments flexible such that it can reversibly bend. In preferred embodiments, the probe is extended and retracted through an opening in the medical device as outlined below.

In a further preferred embodiment of step c) of the computer-aided method according to the invention the movement of said probe with respect to said reference point is determined by:
1) receiving at a first time point a first signal from said medical device which correlates to the distance between the probe and the reference point;
2) receiving at a second time point a second signal from said medical device which correlates to the distance between the probe and the reference point;
3) using the first and second signal received in 1) and 2) to compute said movement.

Said movement can be computed e.g. as the ratio between the distance that the probe moved and the time that has passed during this movement. In this respect the distance moved by the probe is the difference between the first distance obtained in step 1) above and the second distance obtained in step 2) above and the duration of this displacement is the difference between the first and the second time point obtained in steps 1) and 2) above. It is preferred that multiple displacements and/or velocities are computed per second for the probe wherein preferably the time interval between the first and said second time point is maintained constant. To obtain the time points in 1) and 2) above, an electronic timer can be used as is well known in the art.

A tremor is an involuntary movement or shaking of any body part. It is often most noticeable in the hands. Physiological tremor is generally a very-low-amplitude fine tremor (between 6 Hz and 12 Hz) that is barely visible to the naked eye. It is present in every normal individual during maintaining a posture or movement. Fatigue (e.g. rigid muscles), low blood glucose levels, caffeine intake and many other factors influence the frequency and amplitude of fine tremors. If a physician measures the dimensions e.g. of a body cavity such as periodontal pockets, the naturally occurring hand tremor may slightly influence the location of the probe, e.g. the probe may undergo sporadic tiny movements even when the physician makes a conscious effort to not move the probe so that a distance value can automatically be recorded. Thus, in a preferred embodiments of the apparatus and the method of the invention the phrase "has essentially not moved" as used herein above and below means that the maximum displacement of the probe with respect to said reference point within said time interval is smaller or equal than a predefined threshold value. Said predefined threshold value is a value greater than 0 mm. More preferably said predefined threshold value is a value between 0.001mm and 0.8 mm and most preferably the threshold value is a value between 0.001 mm and 0.7 mm, e.g. about 0.024, 0.03, 0.05, 0.1, 0.2, 0.3, 0.4, 0.5, 0.6 or 0.7 mm. The step of determining if said probe has essentially moved is also depicted in item 106 of figure 2 below.

As used herein "has essentially not moved" can also mean that the average velocity of movement of the probe within said time interval is smaller than a predefined threshold value. In this context said predefined threshold value is preferably a value between 0.001 mm/sec and 0.8 mm/sec and most preferably the threshold value is about 0.3, 0.4 or 0.5 mm/sec.

Means to measure hand tremor magnitudes and velocities are well known in the art and such measurements can be carried out without undue burden (see e.g. M. Sälzer, European Journal of Applied Physiology, vol 32, no. 1, march 1973 and Caligiuri and RM Tripp, "A portable hand-held device for quantifying and standardizing tremor assessment", Journal of Medical Engineering & Technology, vol. 28, no. 6. (2004), pp. 254-262).

It is further preferred that in the computer-aided method according to the invention the medical device comprises an opening through which the probe can be extended and/or retracted. Preferably, said opening is said reference point. Thus, it is further preferred that said medical device comprises a reference probe with an opening through which the probe can be extended and/or retracted.

It is further preferred that said medical device comprises a shaft preferably made from a dielectric material, wherein the shaft comprises said opening. Preferably the materials that the medical device and/or shaft is made from consists or comprises an electrically insulating material such as PTFE, PE and PP.

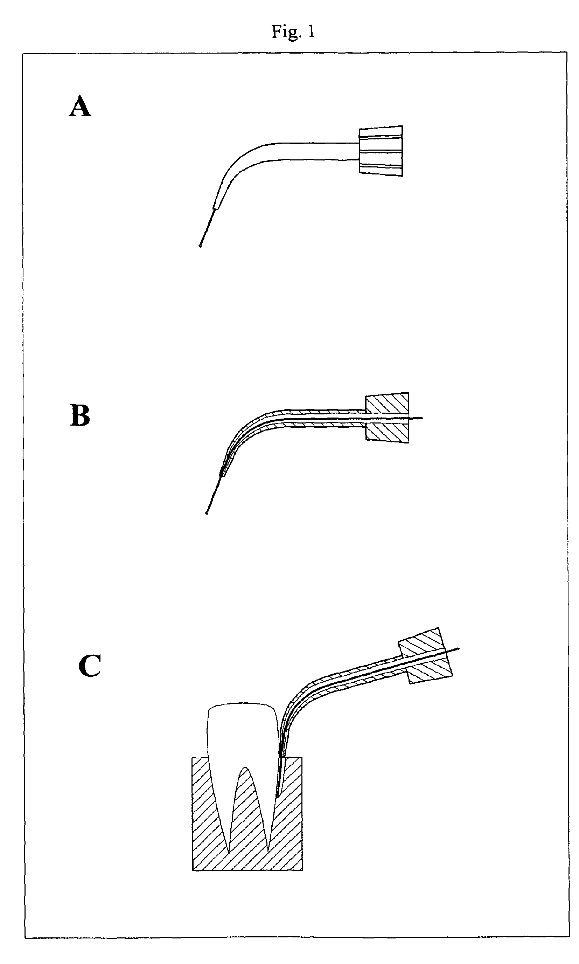

In a preferred embodiment of said shaft, the shaft comprises a rear section and a front section set at an angel of between 90° and 130°, most preferably having an angle as shown in figure 1.

Preferred probes useful for the method, use and apparatus of the invention are elongated and preferably cylindrical probes. Preferably, the probe used according to the invention is elongated and has in its fully extended state a length of not more than 10 mm, 12 mm, 14 mm, 16 mm, 18 mm, 20 mm, 25 mm, 30 mm or not more than 35 mm. Most preferably the length of the elongated probe in its fully extended state measured from the opening in the medical device to the tip of the elongated probe is between 3 mm and 12 mm. In one preferred embodiment the probe is made of stainless steel, surgery steel, copper, silver or mixtures thereof. Further preferred are elongated probes that have a diameter of between 0,1 mm and 0,5 mm preferably between 0,1 to 0,25 mm, e.g. about 0,1 mm, 0,15 mm, 0,2 mm or 0,25 mm. In one preferred embodiment, the probe is elongated and the tip of the elongated probe is configured as a bead with a diameter of between 0,2 mm and 1,0 mm, preferably of between 0,4 mm and 0,6 mm and most preferably of about 0,5 mm. Most preferably the probe is made from a material that is an electrical conductor.

In the following, preferred embodiments of the computer-aided method of the invention will be described that are useful to determine the maximal depth or maximal length of a body cavity.

Thus, in one preferred embodiment of the method of the invention, the medical device comprises an opening through which the probe can be extended and/or retracted and step b) further comprises the steps of:
i) inserting the extended probe into a body cavity under manual control until the tip of the probe touches the distal end of the body cavity;
ii) while maintaining contact between the tip of the probe and said distal end of the body cavity retracting the probe under manual control until the opening in said medical device contacts the opening of the body cavity; and
iii) not moving said probe with respect to said reference point for at least said predefined time interval.

Also preferred is the computer-aided method, wherein the medical device comprises an opening through which the probe can be extended and/or retracted and wherein step b) further comprises the steps of:
i) contacting the opening of a body cavity with the opening of said medical device under manual control;
ii) extending the probe under manual control from the medical device until the tip of the probe touches the distal end of the body cavity; and
iii) not moving said probe with respect to said reference point for at least said predefined time interval.

Thus, in this embodiment, the front end of the medical device, e.g. a shaft as described herein, is rested on the edge of the periodontal pocket, and force is applied, directly or indirectly causing the probe to emerge or extend from the medical device and enter further into the pocket/cavity until the tip of the probe contacts the bottom of the pocket/cavity.

Preferably, said body cavity is selected from the group consisting of a root canal of a tooth, a periodontal pocket and an organ such as the stomach, intestine, artery, vein and heart and parts thereof.

In a further preferred embodiment of the method of the invention the probe is a needle-type electrode and the distance in step d) is measured by carrying out the steps:
i) retrieving one or more impedance or resistance values from said needle-type electrode; and
ii) determining the relative position of the needle-type electrode with respect to said reference point on the basis of said impedance or resistance value(s);
   and wherein the movement of the probe in step c) with respect to said reference point is measured by the further step of:
iii) computing the probe movement from a change in relative positions determined in ii)

Methods wherein a needle-type probe is used in conjunction with the retrieval of one or more impedance or resistance values from said needle-type electrode are comprised in the art and can be implemented by the average skilled person without undue burden. Such methods are described in e.g. US 5,049,069 and US 5,096,419.

In one preferred embodiment of the method of the invention, the method automatically records the distance in step d) only if the condition(s) in step (c) are fulfilled, if said distance is a positive value and if furthermore the operator of the medical device voices an audible command. Methods for voice recognition suitable to be implemented in this preferred embodiment of the method of the invention are well known in the art and can be used by the average skilled person without undue burden (see e.g. US 2009/052636, WO 2009/025356 and US 2009/049610 ).

In a further preferred embodiment of the computer-aided method of the invention, steps b) through d) are repeated at least three times.

Preferred examples of medicinal applications of the computer-aided method of the invention

Gingiva are part of the soft tissue lining of the mouth. Gingiva surround the teeth in collar-like fashion and provide a seal around them. Healthy gingiva are usually coral pink, but may contain physiologic pigmentation. In most individuals, the gingiva which directly contact the teeth form a shallow linear depression, the free gingival groove. This gingival groove which is located between tooth and gingiva is also termed *sulcus gingivae.* The gingival groove next to healthy teeth typically has an average depth of about 2-3 mm and an average width of approximately 0.5-1 mm. However, improper or insufficient oral hygiene can lead to gingival and periodontal disorders, including periodontitis, gingivitis or pyorrhea, which are major causes for tooth failure. Periodontitis involves progressive loss of the alveolar bone around the teeth and may lead to periodontal pockets and eventually the loosening and subsequent loss of teeth if left untreated. Periodontitis is caused by a convergence of bacteria that adhere to and grow on the tooth's surfaces, along with an overly aggressive immune system response against these bacteria. Symptomatic for early stages of such periodontal disorders is the widening of the gingival groove which gives raise to so-called periodontal pockets.

The periodontal disease index (PDI) is an index used for estimating the degree of periodontitis based on the measurement of teeth for gingival inflammation, pocket depth, calculus and plaque, attrition, mobility and lack of contact (see also: Ramfjord SP. The Periodontal Disease Index (PDI). J Periodontol. 1967; 38: 602-610). The PDI can give an accurate assessment of a person's periodontal status. It can be used as a guide in assessing the need for treatment and for evaluation of the results following treatment.

Conventionally, a standardised dental device recommended by the World Health Organisation (a so-called WHO probe) is used to measure the depth of periodontal pockets. The WHO probe comprises a bead having a diameter of 0,5 mm that is located at the distal end of the probe. For measuring the depth of a periodontal pocket the distal end of the WHO probe is inserted into such a pocket, until the bead abuts the gum tissue at the bottom of the pocket. A couple of optical markings located on the distal end of the probe at different distances from the bead allow a dentist to visually determine how far the distal end of the WHO including the bead extends into the periodontal pocket.

However, to provide an accurate assessment of a person's periodontal status, the gingival groove of several teeth is generally examined multiple times for multiple teeth. For each tooth several measurements are read from the optical markings of the dental probe and dictated to an assistant who notes down each individual distance value. The use of a WHO probe is thus strenuous and time consuming for the dentist and additionally requires the presence of an assistant to record the measurements taken.

Therefore, in a particularly preferred embodiment, the computer-aided method of the invention comprises automatically recording a multiplicity of distance values for a multicity of gingival grooves (preferably at least 6) in step d) and, optionally, calculating in a further step the periodontal disease index (PDI) based on these values. An embodiment of the method of the invention that is particularly suitable for the determination of the PDI has been described above and uses a medical device comprising an opening through which the probe can be extended and/or retracted and wherein step b) further comprises either the steps of:
i) inserting the extended probe into a body cavity under manual control until the tip of the probe touches the distal end of the body cavity;
ii) while maintaining contact between the tip of the probe and said distal end of the body cavity retracting the probe under manual control until the opening in said medical device contacts the opening of the body cavity; and
iii) not moving said probe with respect to said reference point for at least said predefined time interval
   or the following steps:

i) contacting the opening of a body cavity with the opening of said medical device under manual control;
ii) extending the probe under manual control from the medical device until the tip of the probe touches the distal end of the body cavity; and
iii) not moving said probe with respect to said reference point for at least said predefined time interval.

In another example from dental root canal therapy, an instrument, e.g. a reamer or a rasp, is inserted into the canal and the material inside the canal is brought to the surface by lateral and rotary movements. There may be one or more canals present depending on the type of the tooth. The operation may involve considerable difficulty due to curves and divisions in the canals. Generally, the operation has a long duration and is laborious. Thus, it would be preferred to shorten the operation.

After cleaning the canal, a flexible filler substance is placed in the root canal and the canal is subsequently sealed with rigid material. If the root canal is not completely cleaned prior to filling and sealing, material left inside the canal can prevent proper healing. Therefore, the dental instrument must be inserted all the way to the apex of the root canal in order to remove all debris. The apical foramen or apex is that anatomical point where the nerves enter a tooth. If the dental instrument is inserted to deeply, the tool penetrates the jaw tissue causing swelling and unnecessary trauma and pain to the patient. To avoid infection and bacterial complications a certain distance, about 1.5 mm, should be maintained from the apical foramen. Thus, it is essential to precisely determine the location of the apical foramen of the tooth root.

Various methods are comprised in the art for determining the location of the apical foramen. For example, in one method for locating the apex of the tooth root, a first electrode, e.g. in the shape of a needle, is inserted into the root canal and second electrode is placed in contact with the patient's body, typically near the patient's mouth, e.g. at his lip. As the first electrode is moved towards the apex, electrical parameters, e.g. impedance or conductance, are measured. Examples of this method are described, e.g. in the following patents: US 5,049,069 and US 5,096,419. However, to record the measured distance from a point within the root canal of a tooth to the apex of the tooth root, the operator of the measuring device has to operate a switch or has to use a second operator who notes down the visualized distance values, resulting in a lengthy operation and possibly inaccurate measurements.

Thus, in another preferred embodiment of the computer-aided method of the invention the distance value in step d) is the distance from a reference point outside of the root canal to a point within the root canal of a tooth. Preferred embodiments useful for determining the depth of a root canal have been described above and comprise the use of a medical device which includes an opening through which the probe is extended and/or retracted and wherein the opening is preferably said reference point.

In a further aspect the invention provides a computer storage medium comprising a computer-readable program code for causing a data processing system to carry out the computer-aided method of the invention.

Also provided is an apparatus for automatically recording the insertion depth of said probe into a body cavity, wherein the apparatus comprises a computer system, means to carry out the computer-aided method of the invention and said medical device including said probe, preferably one of the preferred probes as described herein above.

In a preferred embodiment, the probe comprised in the apparatus is a needle-type electrode and the apparatus is further capable of measuring the distance between the tip of said probe and the medical device by
a) retrieving one or more impedance or resistance values from said needle-type electrode; and
b) determining the relative position of the needle-type electrode on the basis of said impedance or resistance value(s).

In a further preferred embodiment the the apparatus of the invention comprises a means for selecting the time interval used in step c) of the computer aided method according to the invention (see also item 100 shown in figure 2 below).

In a further aspect the invention provides the use of the computer-aided method according to the invention, or the apparatus according the invention, for determining the size of a body cavity preferably selected from the group consisting of a root canal of a tooth, a periodontal pocket, an organ such as the stomach, intestine, artery, vein, heart and other organs and parts, including diseased parts thereof.

Preferably, the probe applied in the use of the invention is elongated and the distance is the distance between said medical device, preferably an opening in said medical device through which the probe can be extended and retracted and the tip of said elongated probe located outside of and distal to said medical device.

### DESCRIPTION OF THE FIGURES

- **Figure 1A**: An example for the medical device of the invention and a preferred example for the probe of the invention comprising at the distal tip a bead is shown.
- **Figure 1B**: A across section through a preferred embodiment of the medical part of the invention is depicted. As shown in this example, the probe is elongated and can be extended and retracted through an opening in the medical device.
- **Figure 1C**: A preferred use of the method of the invention is shown which is the measurement of the depth of a periodontal pocket.
- **Figure 2**: This figure is a flow chart providing an overview of a preferred embodiment of the computer-aided method of the invention.

The present invention will now be further described using examples and different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### EXAMPLES

### Example 1:

A medical device as shown in figure 1, wherein the probe is an elongated probe in form of a periodontal probe and the medical device is made from plastic can be coupled to an electronic device capable of measuring the distance between the probe and the opening in the shaft of the medical device shown in figure 1. The electronic device is further equipped with an apparatus capable of carrying out the method of the invention and an input device in form of a dial which could be used to set the time interval used in the method of the invention. The extendable and retractable probe was shaped as shown in figure 1, having a diameter of 0,2 mm and having a tip comprising a bead with a diameter of 0,5 mm.

As an example, as the time interval used for the examination which will be described in detail below, a setting of 0,5 seconds can be set. Before each measurement, the probe is extended to a length of 10 mm. Under the application of soft pressure (<0.2 N), the distal end of the probe is inserted into the gingival groove or, if present, into periodontal pockets, until the bead of the probe tip abuts the gum tissue at the bottom of the pocket or groove. During insertion of the probe the bead at the tip of the probe should contact the tooth and follow the anatomical configuration of the surface of the tooth root until the deepest end of the pocket or of the groove is reached.

Next, while keeping contact between the distal tip of the probe and the deepest end of the pocket or groove, the medical device is moved under manual control over the probe and towards the gum tissue (i.e. the probe will be retracted through the opening of the medical device) until the opening in the medical device contacts the gum tissue surrounding the upper opening i.e. the fringe of the periodontal pocket or of the gingival groove of the tooth being examined, respectively. In this preferred embodiment of the method of the invention the reference point is defined to be said opening in the shaft of the medical device, as also shown in fig. 1 C. Once the opening touches the upper opening of the pocket or groove, respectively, the operator waits about one second to trigger the automated distance recording in step d) of the method. After the automatic recording the probe is removed from the groove or pocket and is reset to the initial extension length of 10 mm. The next measurement is taken by repeating the above outlined procedure.

For each tooth a total of 6 distance (pocket and groove) measurements are automatically recorded for 6 points surrounding each tooth (mesio-buccal, mid-buccal, disto-buccal, and the corresponding lingual sites, as also recommended by the WHO: http://www.whocollab.od.mah.se/expl/orhcpitn.html).

After the automatic recording of 6 distance values for each tooth, the apparatus of the invention can suggest another tooth to be examined and the above-described steps are repeated with respect to this second tooth and so on. In total it is recommended to repeat the measurements and the automated recordings of distance values for at least six representative teeth. After all teeth have been examined, the "measured" depths of the periodontal pockets of the teeth that were examined can be stored in a database connected to the electronic apparatus in order to update the patient data.

## Claims

1. A computer-aided method for automatically recording the distance between a probe of a medical device and a reference point comprising the steps of:
a) providing a medical device comprising a probe;
b) moving said probe under manual control with respect to a reference point;
c) automatically determining
(i) if said probe has essentially not moved with respect to said reference point during a predefined time interval and/or
(ii) if the directionality of movement of said probe with respect to said reference point was reversed during a predefined time interval;
and
d) automatically recording the distance between said probe and said reference point if at least one of the conditions in step (c) are fulfilled.

2. The computer-aided method according to claim 1, wherein the localization of said reference point is defined by a reference probe.

3. The computer-aided method according to claims 1 or 2, wherein step c) is carried out during or after step b).

4. The computer-aided method according to any of claims 1-3, wherein in step c) the movement of the probe with respect to said reference point and/or the distance in step d) is measured with an optical, mechanical or electrical means.

5. The computer-aided method according to any of claims 1-4, wherein in step c) the movement of said probe with respect to said reference point is determined by:
1) receiving at a first time point a first signal from said medical device which correlates to the distance between the probe and the reference point;
2) receiving at a second time point a second signal from said medical device which correlates to the distance between the probe and the reference point;
3) using the first and second signal received in 1) and 2) to compute said movement.

6. The computer-aided method according to any of claims 1-5, wherein in step c) (i) said probe has essentially not moved with respect to said reference point during a predefined time interval if:
(a) the maximal displacement of the probe with respect to said reference point within said time interval is smaller or equal than a predefined threshold value and/or
(b) the average velocity of the probe within said time interval is smaller than a predefined threshold value.

7. The computer-aided method according to any of claims 1-6, wherein the medical device comprises an opening through which the probe can be extended and/or retracted.

8. The computer-aided method according to any of claims 1-7, wherein the probe is elongated and has in its fully extended state a length of not more than 35 mm.

9. The computer-aided method according to any of claims 1-8, wherein the medical device comprises an opening through which the probe can be extended and/or retracted and wherein step b) further comprises the steps of:
i) inserting the extended probe into a body cavity under manual control until the tip of the probe touches the distal end of the body cavity;
ii) while maintaining contact between the tip of the probe and said distal end of the body cavity retracting the probe under manual control until the opening in said medical device contacts the opening of the body cavity; and
iii) not moving said probe with respect to said reference point for at least said predefined time interval.

10. The computer-aided method according to any of claims 1-8, wherein the medical device comprises an opening through which the probe can be extended and/or retracted and wherein step b) further comprises the steps of:
i) contacting the opening of a body cavity with the opening of said medical device under manual control;
ii) extending the probe under manual control from the medical device until the tip of the probe touches the distal end of the body cavity; and
iii) not moving said probe with respect to said reference point for at least said predefined time interval.

11. The computer-aided method according to claims 9 or 10, wherein said body cavity is selected from the group consisting of a root canal of a tooth, a periodontal pocket, an organ and parts thereof.

12. A computer storage medium comprising a computer-readable program code for causing a data processing system to carry out the computer-aided method of any of claims 1 to 11.

13. An apparatus for automatically recording the insertion depth of said probe into a body cavity, wherein the apparatus comprises a computer system, means to carry out the computer-aided method of any of claims 1 to 11 and said medical device including said probe.

14. The apparatus according to claim 13, wherein in step c) of the method performed by the apparatus the movement of the probe with respect to said reference point and/or the distance in step d) is measured with an optical, mechanical or electrical means.

15. Use of the computer-aided method according to any of claims 1-11, or the apparatus according to claims 13 or 14, for determining the size of a body cavity, of an organ or parts thereof.
